(19) Europäisches Patentamt

European Patent Office

Office européen des brevets

(11) EP 0 689 459 B1

(12) EUROPEAN PATENT SPECIFICATION

(45) Date of publication and mention
of the grant of the patent:
04.12.2002 Bulletin 2002/49

(51) Int Cl.⁷: **A61K 47/48**, C07K 14/33,
C12N 15/62

(21) Application number: 94909262.1

(22) Date of filing: 18.03.1994

(86) International application number:
PCT/GB94/00558

(87) International publication number:
WO 94/021300 (29.09.1994 Gazette 1994/22)

(54) **NOVEL AGENT FOR CONTROLLING CELL ACTIVITY**

MITTEL FÜR DIE STEUERUNG VON DER ZELLAKTIVITÄT

NOUVEL AGENT DE REGULATION DE L'ACTIVITE CELLULAIRE

(84) Designated Contracting States:
AT BE CH DE DK ES FR GB GR IE IT LI LU NL PT
SE

(30) Priority: 19.03.1993 GB 9305735

(43) Date of publication of application:
03.01.1996 Bulletin 1996/01

(73) Proprietors:
• **THE SPEYWOOD LABORATORY LTD.**
**Slough, Berkshire SL1 3XE (GB)**
• **MICROBIOLOGICAL RESEARCH AUTHORITY**
**Salisbury, Wiltshire SP4 0JG (GB)**

(72) Inventors:
• **NORTH, John Robert**
**Amesbury Wilts. SP4 7EU (GB)**
• **FOSTER, Keith Alan**
**Surrey KT4 8XG (GB)**
• **QUINN, Conrad Padraig**
**Wilts. SP1 3QS (GB)**
• **SHONE, Clifford Charles**
**Wilts. SP5 3BN (GB)**

(74) Representative: **MacLean, Martin Robert et al**
**Mathys & Squire**
**100 Gray's Inn Road**
**London WC1X 8AL (GB)**

(56) References cited:
WO-A-91/17173          WO-A-92/00099
WO-A-93/04191

• **BIOCHEMISTRY, vol.33, 1994, EASTON, PA US**
**pages 2604 - 2609 SIMS K. KOCHI ET AL. 'THE**
**EFFECTS OF PH ON THE INTERACTION OF**
**ANTHRAX TOXIN LETHAL AND EDAMA**
**FACTORS WITH PHOSPHOLIPID VESICLES'**

**Description**

TECHNICAL FIELD

This invention describes a novel agent for the targeted control of mammalian cell activity, in particular the agent is used to control the interaction of particular cell types with their external environment. The agent has applications as a pharmaceutical for the treatment of a variety of disorders.

BACKGROUND

A fundamental property of living cells is their ability to respond to their external environment. The interface between a cell and its external environment is the plasma membrane. The plasma membrane consists of a phospholipid bilayer in which many kinds of protein molecules are embedded. These integral membrane proteins (IMPs) are responsible for many of the interactions of a cell with its external environment.

The interactions in which the IMPs are involved include: the transport of materials, including nutrients, into and out of the cell; the regulated permeability of the plasma membrane to ions; the recognition of, and response to, extracellular molecules; and the adhesion of one cell to another cell. A specialised function of the immune system, that is also mediated via IMPs, is the display of particular foreign peptide sequences by one group of immune cells to another group.

One of the ways in which a cell regulates its ability to respond to, and interact with, the external environment is by changing the quantity and types of IMPs present at the plasma membrane. One mechanism by which this is achieved is the reversible internalisation of IMPs via an endocytotic pathway into Recyclable Membrane Vesicles (RMVs). In these cases IMPs stored in the RMVs represent an internal store or pool of IMPs available for rapid export to the cell surface via a process of exocytotic fusion of the RMVs with the plasma membrane. Modulation of the equilibrium of this exocytotic / endocytotic cycle allows rapid regulation of the density of IMPs present at the cell surface. In one example of the process for controlling cell activity, the uptake of glucose by insulin-responsive cells in skeletal muscle and adipose tissue is regulated. Insulin increases the amount of a particular isoform of glucose transporter, GLUT4, which is found in the plasma membrane of these cells. The higher concentration of GLUT4 molecules at the surface of the cell results in increased uptake of glucose. Therefore, by controlling the number of glucose transporters present in the plasma membrane the response to insulin can be modulated.

Another example of alterations in cell surface IMP expression in response to external signals is that of the receptor for the complement fragments C3b and C4b, the type 1 complement receptor CR1. Upon activation of human neutrophils the plasma membrane expression of CR1 is transiently increased 6- to 10- fold.

In another example a number of inflammatory and immune cells modify their expression of cell surface adhesion molecules upon activation. Hence, activation of neutrophils or monocytes leads to a modulation of the cell surface adhesion molecules Mac-1 and p150,95. These adhesion molecules are important in the targeting and movement of inflammatory cells to sites of inflammation.

In yet another example, a variety of hormones (insulin, insulin-like growth factor, interleukin 1 and platelet-derived growth factor) cause a rapid increase in the cell surface expression of the transferrin receptor in a variety of cell types. The transferrin-receptor binds diferric transferrin from the external environment of the cell, and is thereby involved in the uptake of iron by cells. This transferrin/transferrin-receptor system may also play a role in the transcellular movement of iron into the CNS across the blood brain barrier, a process known as transcytosis. Transcytosis is also involved in the transfer of maternal immunity to the developing foetus.

In yet another example the diuretic hormone aldosterone is known to increase the cell surface expression of $Na^+$ channels in the apical membrane of urinary bladder epithelial cells. This mechanism is involved in salt retention and occurs, for example, in conditions of low sodium-containing diets.

In a further example of the modulation of cell membrane expression of IMPs, it is noted that the function of the immune system is based upon the recognition of foreign, or non-self, antigens. Part of this recognition and immune response is provided by cells of the immune system able to recognise and respond to foreign peptide sequences. These peptide sequences are presented to the immune cells by other cells of the immune system known as antigen presenting cells. Antigen presenting cells ingest foreign antigenic proteins, digest these to peptides and display the foreign peptides in a cleft formed at the cell membrane by IMPs of the major histocompatibility complex.

Thus IMPs are central to a cell's ability to interact with its external environment and, given the diverse and varied nature of these interactions, it is not surprising to discover that there are a vast array of different IMPS. The pivotal role of IMPs in a cell's function means that they are often involved in pathophysiologic states, and are the target for many therapeutic interventions.

Prior art approaches to the control of IMP activity have mainly focused on modulating the function of the IMP once expressed at the cell surface. Thus prior art therapeutic interventions tend to be specific for particular IMPS and for particular functions of particular cell types. Inhibitors of specific transport IMPs have been developed as therapeutic agents. For example, inhibitors of the 5HT transport protein of neurones are used as anti-depressants. Antagonists of particular receptor IMPs are very commonly used pharmaceutical agents. Examples include antihistamines, both those specific for the H1 and the H2 subtypes of histamine receptor, and antagonists of the β-adrenoceptor. Inhibitors of IMP function are also widely used as pharmaceutical agents. Examples include inhibitors of transmembrane ion movements such as the diuretics furosemide and amiloride, the latter of which is an inhibitor of the bladder epithelial cell apical $Na^+$ channel. Inhibitors of potassium channels are known to be under development as antiarrhythmic agents. Cell adhesion IMPs are also currently targets for the development of selective antagonists.

Another approach being pursued is to selectively modify the expression of particular IMPs at the genetic level by alteration of the level of transcription of the appropriate gene coding for that IMP and hence modulation of specific IMP protein synthesis.

In summary, IMPs are known to play a critical role in the response of a cell to its external environment. Previous approaches to the control of IMPs have generally involved the targeting of a specific IMP at the cell surface and modifying its functional capacity. The control of the density of IMPs within the plasma membrane is anticipated to have broad applications in the treatment of a variety of disorders. In view of the great diversity of IMPs and the particular nature of current therapeutic interactions it is the surprising discovery of the current invention that a single class of agents can modify the expression of IMPs in a wide variety of cell types. The same class of agent is also able to modify the expression of transport IMPs, receptor IMPs, adhesion IMPs, channel IMPs and antigen presenting IMPs. Previously, agents affecting IMPs have been classified by function, for example $Ca^{++}$ antagonists, the members of each group being chemically and mechanistically very diverse. The class of agent referred to in the current invention, by contrast, is structurally homogeneous, with rationally introduced substitutes of particular domains having predictable effects on the function of the agent. A further aspect of the invention is that the agent can be selectively targeted to particular types of cell to allow selective modulation of IMP expression only in that cell type.

STATEMENT OF INVENTION

The current invention relates to an agent for controlling the interaction of a cell with its external environment. Specifically, the invention provides an agent for controlling the transport of Integral Membrane Protein (IMP) molecules from the internal components of a cell to the cell membrane, so as to modify the cell's interaction with its external environment. More specifically the invention provides a novel agent which modifies the structure of Recyclable Membrane Vesicles (RMVs) such that their ability to transport IMPs to the surface of the cell is inhibited. The invention is further defined in the claims.

Definitions

The following terms have the following meanings;

Integral Membrane Protein (IMP) means any protein which is embedded in and spans across the lipid bilayer of a biological membrane

Recyclable Membrane Vesicle (RMV) means an intracellular vesicle, present in the cytosol of a cell, bounded by a lipid bilayer membrane . RMVs are formed from the plasma membrane and move into the cell interior by a process referred to as endocytosis. RMVs undergo a cyclical process of forming from and fusing with the cell plasma membrane. The process of moving to and fusing with the plasma membrane is referred to as exocytosis. The function of RMVs in the cell is in the reversible transport of IMPS to and from the cell surface; in this they are distinct from the secretory vesicles of neurosecretory cells.

Endosome means those intracellular vesicles which have formed from the plasma membrane by a process of endocytosis.

Heavy chain means the larger of the two polypeptide chains which form Clostridial neurotoxins; it has a molecular mass of approximately 100 kDa and is commonly referred to as HC. Light chain means the smaller of the two polypeptide chains which form Clostridial neurotoxins; it has a molecular mass of approximately 50 kDa and is commonly referred to as LC. Naturally occurring Heavy and Light chains are covalently coupled via at least one disulphide bond.

$H_2$ fragment means a fragment derived from the amino terminal end of the Heavy chain of a Clostridial neurotoxin by proteolytic cleavage for example with trypsin or papain.

$H_2L$ means a fragment of a Clostridial neurotoxin produced by proteolytic cleavage for example with trypsin or papain in which the Light chain is still coupled via disulphide bonds to the $H_2$ fragment.

In one aspect of the invention an agent is provided for the control of the level of the IMP responsible for the transport of a metabolite across the cell membrane, so controlling the availability of that metabolite within the cell.

In another aspect of the invention an agent is provided for the control of the level of the IMP responsible for the transport of a metabolite across the cell membrane into the cell and out of the cell, so controlling the transport of that metabolite through the cell.

In yet another aspect of the invention an agent is provided for the control of the level of the IMP responsible for the selective permeability of the plasma membrane of the cell to an ion, so modulating the concentration of that ion within the cell.

In yet another aspect of the invention an agent is provided for the control of the level of the IMP responsible for the recognition of a signalling molecule, so modulating the responsiveness of the cell to that signalling molecule.

In yet another aspect of the invention an agent is provided for the control of the level of the IMP responsible for the transduction of signals across the cell membrane following binding to the membrane of a signalling molecule, so modulating the responsiveness of the cell to that signalling molecule.

In yet another aspect of the invention an agent is provided for the control of the level of the IMP responsible for the display on the cell surface of peptide fragments derived from ingested antigens. The result of this in an organism is to affect the immune response of that organism.

The invention also provides an agent which has target specificity for target cell types so that the scope of the effect of the agent is limited to said cell types.

As previously stated, prior art approaches to the control of IMP activity have mainly focused on modulating the function of the IMP <u>once expressed</u> at the cell surface. In direct contrast the present invention modulates the level of IMP which <u>becomes expressed</u> at the cell surface.

<u>DETAILED DESCRIPTION OF THE INVENTION</u>

It can be seen that the object of this invention, to provide an agent for controlling the level of IMPs at a cell surface, has many potential applications for modulating the response of a cell to its environment. This invention includes an agent which functions so as to affect the mechanisms by which IMPs are carried to the surface of a cell, as evidenced in the examples, e.g. example 1 and 2. Such an agent must accomplish three discrete functions, the first two of which are known in the art. Firstly it must bind to a cell surface structure (the Binding Site). It must then enter into the cytosol of the cell. The entry of molecules into the cell is known to occur by a process of endocytosis. However, as only certain cell surface Binding Sites are known to be involved in endocytosis, only these Binding Sites are suitable as targets. Once taken into the cell by endocytosis the agent must then leave the resulting endosome across the endosomal membrane to enter the cytosol. The ability to achieve specific cell binding and entry of agents into the Cytosol is well known in the literature (for example: Pastan, I Willingham, MC; & Fitzgerald, DSP, 1986, Cell <u>47</u> 641 - 648, Olsnes, S; Sandvig, K; Petersen, OW; & Van Dews, B, 1989, Immunol. Today <u>10</u>, 291 - 295, Strom, TB; Anderson, PL; Rubin-Kelley, VE; Williams, DP; Kiyokawa, T; & Murphy, JR; 1991, Ann NY Acad. Sci <u>636,</u> 233 - 250). The third function of the agent is the surprising finding of this invention, namely the ability to affect the RMV. The further surprising aspect of this agent is that by so affecting the RMV it limits its ability to transport the IMPs to the cell surface.

The agent of this invention therefore comprises the following functional Domains;

Domain B, the Binding Domain, binds the agent to a Binding Site on the target cell capable of undergoing endocytosis to produce an endosome containing the agent, wherein Domain B is not the native Binding Domain of a Clostridial neurotoxin

Domain T, the Translocation Domain, translocates Domain E (with or without other domains of the agent and/or the Binding Site) from within the endosome across the endosomal membrane into the cytosol of the cell

Domain E, the Effector Domain, inhibits transport of IMPs to the surface of the cell by RMVs, and is a domain or domain fragment of the Light chain of a Clostridial neurotoxin having $Zn^{2+}$ dependent metalloprotease activity;

with the proviso that the agent is not the disulphide-linked conjugate $I_{bc}$-ricin B chain or the disulphide-linked conjugate $I_{bc}$-wheat germ agglutinin, wherein $I_{bc}$ is obtained by enzymic treatment of tetanus toxin with papain.

Domain B can be made to have specificity for a target cell type. The ability to target an agent to a particular cell type is well known in the art. Thus, the functions of Domain B could be achieved by the use of one of many cell-binding molecules known in the art including, but not limited to, antibodies, monoclonal antibodies, antibody fragments (Fab, F(ab) '$_2$, Fv, single chain antibodies, etc.), hormones, cytokines, growth factors and lectins.

The functions of Domain T could be achieved by molecules capable of forming appropriate pores within the endosomal membrane. It is well documented that certain parts of toxin molecules are capable of forming such pores including, amongst others, fragments of anthrax toxin, botulinum toxin, tetanus toxin, Diphtheria toxin and Pseudomonas endotoxin (Hoch, DH; Romero-Mira, M; Ehrlich, BE; Finkelstein, A; Das Gupta, BR; & Simpson, LL; 1985 PNAS 82 1692 - 1696, Olsnes, S; Stenmark, H; Moskaug, JO; McGill, S; Madshus, IH; & Sandvig, K, 1990, Microbial Pathogenesis 8, 163 - 168.) One such molecule is the Heavy chain of clostridial neurotoxins for example botulinum neurotoxin type A. Preferably it has been found to use only those portions of the toxin molecule capable of pore-forming within the endosomal membrane.

The functions of Domain E, the inhibition of the ability to transport the IMPs to the surface of the cell are not known to the art. Surprisingly, it has been found that different portions of certain toxin molecules - functionally distinct from those capable of pore-formation, including fragments of clostridial neurotoxins, such as either botulinum or tetanus toxins, when introduced into the cytoplasm of target cells are capable of inhibiting the transport of the IMPs in RMVs to the surface of the cell, so reducing the concentration of those IMPs at the cell surface. In particular it has been found that fragments of tetanus toxin and botulimum types A, B, C$_1$, D, E, F and G are particularly suitable. In a preferred embodiment, Domain E is the Light chain of the botulinum neurotoxin type B having Zn$^{++}$ dependent metalloprotease activity.

The invention therefore includes an agent of the following structure;

Domain B-Domain T-Domain E;

with the proviso that the agent is not the disulphide-linked conjugate I$_{bc}$-ricin B chain or the disulphide-linked conjugate I$_{bc}$-wheat germ agglutinin, wherein I$_{bc}$ is obtained by enzymic treatment of tetanus toxin with papain.

The Domains are covalently linked by linkages which may include appropriate spacer regions between the Domains.

In one embodiment of the invention Domain B is a binding molecule capable of binding to a Binding Site on the target cell which undergoes endocytosis, Domain T is the Heavy chain of a botulinum neurotoxin or fragments thereof and Domain E is the Light chain of a botulinum neurotoxin or fragments thereof. Domains T and E can be from the same or different serotypes of *C.botulinum.*

In another embodiment of the invention Domain B is a binding molecule capable of binding to a Binding Site on the target cell which undergoes endocytosis, Domain T is the Heavy chain of a tetanus neurotoxin or fragments thereof and Domain E is the Light chain of a botulinum neurotoxin or fragments thereof.

In another embodiment of the invention Domain B is a binding molecule capable of binding to a Binding Site on the target cell which undergoes endocytosis, Domain T is the Heavy chain of a botulinum neurotoxin or fragments thereof and Domain E is the Light chain of a tetanus neurotoxin or fragments thereof.

In another embodiment of the invention Domain B is a binding molecule capable of binding to a Binding Site on the target cell which undergoes endocytosis, Domain T is the Heavy chain of a tetanus neurotoxin or fragments thereof and Domain E is the Light chain of a tetanus neurotoxin or fragments thereof.

It is to be understood that this invention includes any combination of toxin molecules or fragments of toxin molecules from the same or different organisms which have the functions described.

When the agent is administered to an organism the concentration of IMPs at the surface of the target cell is reduced. This can lead to a number of desired effects including reduced intake of a metabolite or ion into or across the cell, reduction in response of the target cell to a signalling molecule, or change in the immune response of the organism.

EXAMPLES

EXAMPLE 1

3T3-LI fibroblasts are trypsinized into suspension and are electroporated at 300V/ cm, 960 mF with a time constant of 11 - 11.5 msecs, using a Bio-Rad Gene Pulser with capacitance extender, in the presence or absence of 1μM botulinum neurotoxin-B (BoNT-B). Following electroporation the cells are allowed to adhere and are maintained in monolayer culture at 37°C in 24-well plates for 72 h. The cells are then washed and incubated for 5 min at 37°C in the presence or absence of 5nM insulin-like growth factor type 1 (IGF-1), followed by standing on ice for 5 min. The supernatant is aspirated from the cells and replaced with ice-cold 1.5 nM $^{125}$I-transferrin (sp. act. 47 Tbq / mmol). Non-specific binding is estimated in parallel incubations performed in the presence of a 100 fold molar excess of non-radioactive transferrin. After 2h the supernatant is removed, and following 3 washes with ice-cold buffer the cell layer is digested in 1N NaOH, and the bound $^{125}$I-transferrin measured using a LKB1275 minigamma gamma counter. Up-regulation of transferrin-binding is calculated as the specific $^{125}$I-transferrin binding in the presence of IGF -1 expressed as a percentage of the specific binding in the absence of IGF-1.

Table 1 shows that there is a reduced elevation of $^{125}$I-transferrin binding in response to IGF-1 in BoNT-B treated cells compared to control. This indicates that introduction of BoNT-B into the cytosol of 3T3-LI fibroblasts inhibits the IGF-1 stimulated up-regulation df transferrin receptors in these cells.

Triton-X-114-soluble proteins extracted from the 3T3-LI fibroblasts digests are analysed by Western blotting using a polyclonal antibody raised against a peptide sequence SEQ.ID.1: (QQTQAQVDEVVDIMRVNVDKVLERDQKLSELDDRADALQAGASQFETSAAKLKRKYWWK NLK) identified in a secretory vesicle protein of neurosecretory cells. This anti-vesicle antibody shows reduced reactivity with the relevant doublet band in samples from BoNT-B-treated fibroblasts, which have no reported neurosecretory activity. Thus, BoNT-B is modifying vesicle (presumably RMV) structure in 3T3-LI fibroblasts concurrently with inhibiting up-regulation of transferrin receptors.

EXAMPLE 2

3T3-LI fibroblasts are electroporated in the presence or absence of 0.5 $\mu$M botulinum neurotoxin-A (BoNT-A) using conditions identical to those given in example 1. IGF-1 stimulation of $^{125}$I-transferrin binding is assayed in treated and untreated cells as described in example 1.

The results in table 2 show that BoNT-A treatment of 3T3-LI fibroblasts abolishes the up regulation of $^{125}$I-transferrin binding seen in response to IGF-1. This indicates that introduction of BoNT-A into the cytosol of 3T3-LI fibroblasts inhibits the IGF-1 stimulated up-regulation of transferrin receptors in these cells.

EXAMPLE 3

3T3-LI fibroblasts are electroporated in the presence or absence of 0.5$\mu$M of the $H_2$L-fragment of BoNT-A ($H_2$L-A) using conditions identical to those given in example 1. This fragment is produced from the neurotoxin, serotype A, of C botulinum by limited proteolysis using tosylphenylalaninechloromethane-treated trypsin. The $H_2$L complex is then purified by chromatography on Sephadex G-200 (Shone, CC; Hambleton, P; & Melling, J; 1985, Eur J Biochem 151, 17-82). Electroporation is performed as described in example 1 as is the measurement of IGF-1 stimulation of $^{125}$I-transferrin binding in treated and untreated cells.

The results in table 3 show that $H_2$L-A treatment of 3T3-LI fibroblasts inhibits the up-regulation of $^{125}$I-transferrin binding seen in response to IGF-1. This indicates that introduction of the $H_2$L-A fragment of botulinum neurotoxin-A into the cytosol of 3T3-LI fibroblast inhibits the IGF-1 stimulated up-regulation of transferrin receptors in these cells.

EXAMPLE 4

3T3-LI adipocytes are differentiated from 3T3-LI fibroblasts by treatment with dexamethasone, 3-isobutyl-1-methylxanthine and insulin as described (Frost, SC & Lane MD, 1985, J Biol Chem 260, 2646 -2652). The 3T3-LI adipocytes 7 days after differentiation are treated with Botulinum neurotoxin serotype A diluted into Dulbecco's modified Eagles medium containing serum and filter sterilised (final concentration BoNT A:200nM). Toxin treated and control cells are incubated at 37°C for 45 hours in 8% $CO_2$. The cells are then washed twice and incubated in 8% $CO_2$ for 2 hours in serum-free Dulbecco's modified Eagle's medium after which the cells are washed in Krebs Ringer phosphate and incubated in either Krebs Ringer phosphate (basal uptake) or Krebs Ringer phosphate containing 100nM insulin (stimulated uptake) for 15 minutes at 37°C. Glucose uptake is initiated by the addition of [3H] 2-deoxyglucose (14.2KBq, 10uM glucose). After 10 minutes at 37°C the reaction is stopped by aspiration of the glucose solution and rapid washing with ice cold phosphate buffered saline. Cells are lysed by the addition of 0.2N NaOH and the solution neutralised by the addition of 0.2N HCl. Uptake of [3H] 2-deoxyglucose is measured by liquid scintillation counting in optiphase scintillant using a Wallac 1410 liquid scintillation counter.

It is known that clostridial neurotoxins are able to enter certain neurosecretory cells (for example PC12 cells) via a low affinity receptor if high concentrations of the neurotoxin are incubated with the cells for prolonged periods. This process appears to use a pathway via a receptor which is distinct from the highly specific and high affinity receptor present at the neuromuscular junction. Additionally it has been reported that certain clostridial toxins have effects on phagocytic cells, such as macrophages, where entry into the cell is presumed to be via the specific phagocytic activity of these cells. Generally, it is recognised, however, that the neuronal selectivity of clostridial neurotoxins is a result of a very selective binding and cell entry mechanism. It is, therefore, selective binding and cell entry mechanism. It is, therefore, the surprising'finding of these studies, that incubation of 3T3-LI adipocytes with botulinum neurotoxin-A, as described, causes a marked inhibition of insulin-stimulated up-regulation of [3H] 2-deoxyglucose transport (table 4). It is known that insulin-up regulation of glucose transport in adipocytes is a result of movement of glucose transporter proteins from intracellular pools (RMVs) to the cell surface. Thus, this result demonstrates that botulinum neurotoxin-A inhibits the insulin-stimulated movement of glucose transporters in RMVs to the cell surface of adipocytes.

EXAMPLE 5

3T3-LI adipocytes are trypsinised and a suspension of the cells is electroporated in the presence or absence of Botulinum B ($0.32\mu$M). A 960 mF capacitor is used for electroporation producing a pulse strength of 300 V/cm; the time constant is 11-12 ms. After electroporation the cells are washed and plated out in a 6 well plate with media and serum. The cells are incubated at $37^{\circ}$C in a humidified atmosphere (air/$CO_2$; 92.5%/7.5%) for 72 h. At the end of this period, the cells are washed and extracted into 0.1N NaOH. Following neutralisation of the extract with 0.1N HCl the membrane proteins are partitioned into Triton X-114 and subsequently analysed by Western blotting using the anti-vesicle antibody described in example 1. The surprising finding of this study is that electroporation of botulinum neurotoxin into the cytosol of adipocytes results in a modification of vesicle (presumably RMV) structure as evidenced by reduced reactivity of the antibody with the relevant doublet band on samples from botulinum neurotoxin-B treated cells.

EXAMPLE 6

In this example, an agent is synthesized to regulate the cell surface expression of the insulin-dependent glucose transporter of adipocytes.

The binding Domain (B) for the agent in this example is insulin-like growth factor II, which is purified from the conditioned medium of BRL-3A cells as described (Marquette, H; Todaro, GJ; Henderson, LE & Oroszlan, S, 1981, J Biol. Chem 256 2122-2125).

The translocating Domain (T) is prepared from the neurotoxin, serotype A, of C. *botulinum* by limited proteolysis of the neurotoxin with tosylphenylalaninechloromethane-treated trypsin. The fraction containing Domain T is then purified by chromatography on Sephadex G-200 (Shone, CC; Hambleton, P; & Melling, J; 1985, Eur. J. Biochem. 151, 75-82). This fraction is then applied in phosphate/borate buffer, pH 8.4 onto a quaternary aminoethyl-Sephadex column, and incubated on the column at $4^{\circ}$C overnight with 2M urea and 0.1M dithiothreitol. The column is then washed with buffer containing 2M urea and10mM dithiothreitol. Domain T is then eluted using phosphate/borate buffer containing 2M urea and 10mM dithiothreitol and a stepwise gradient of NaCl from 0.1 to 0.2M (Poulain, B; Wadsworth, JDF; Maisey, EA; Shone, CC; Melling, J; Tauc, L; & Dolly, JO, 1989, Eur. J. Biochem. 185, 197-203). The clostridial neurotoxins are disulphide-linked dichain proteins consisting of a heavy chain and a light chain (Simpson, LL, 1986, Ann. Rev. Pharmicol. Toxicol. 26, 427-453). It should be noted that the Domain T, produced in the manner given, is equivalent to a fraction of the heavy chain of the neurotoxin referred to as $H_2$.

The effector Domain (E) is prepared from the neurotoxin of C. tetani by isoelectric focusing in a sucrose gradient with ampholyte under reducing conditions in 2M urea (Weller, U; Dauzenroth, M-E; Meyer zu Heringdorf, D & Habermann, E, 1989, Eur. J. Biochem., 182, 649-656). It should be noted that the Domain E produced in the manner given is equivalent to the light chain of the neurotoxin, commonly referred to as LC.

Domains E and T are mixed together in equimolar proportions under reducing conditions and covalently coupled by repeated dialysis, at $4^{\circ}$C with agitation, into physiological salt solution in the absence of reducing reagents. Any remaining free sulphydryls are derivatized by the addition of 150mM iodoacetamide for 30 min at $4^{\circ}$C in the dark. The conjugated E-T product is purified by size exclusion chromatography on Sephadex G-150 using potassium phosphate buffer, pH 7.0. Finally, Domain B is coupled to the E-T complex using N-succinimidyl 3-(2-pyridylothio) proprinate (SPDP). The E-T complex (5 mg) is dissolved in 1 ml of phosphate buffered saline (PBS), and to this is added 200 mg of SPDP dissolved in 0.5 ml of absolute ethanol. After reacting the mixture at room temperature for 30 mins, the 2-pyridyldisulphide-substituted peptide is separated from excess SPDP by gel filtration through Sephadex G25. Domain B is similarly treated, but using less SPDP (20 mg in 0.2 ml ethanol). The substituted Domain B is again harvested from a Sephadex G25 column, and is then reduced by the addition of dithiothreitol to a final concentration of 0.05M. Excess reducing agent is removed by gel filtration on Sephadex G25. Equal portions (w/w) of the substituted E-T complex and the substituted and reduced Domain B are then mixed together and left at $4^{\circ}$C for 18h. The agent is then purified by chromatography on Sephadex G-150 using potassium phosphate buffer, pH 7.0.

The agent, prepared as described, is then tested for its ability to inhibit the insulin-stimulated increase in glucose transporter expression in 3T3-L1 adipocytes. 3T3-L1 adipocytes are differentiated from 3T3-L1 fibroblasts by treatment with dexamethasone, 3-isobutyl-1-methylxanthine and insulin as described (Frost, SC & Lane MD, 1985, J Biol Chem 260, 2646-2652), and are used between 8 and 12 days after initiation of differentiation. Cells are incubated with or without the agent for 90 min at $37^{\circ}$C. Cells are then incubated for 2 hours in serum-free Dulbecco's modified Eagle's medium at the beginning of each experiment. Insulin-treated cells are then exposed for 10 minutes to $10^{-7}$M insulin which is added from a stock 1.6 x $10^{-4}$ M solution. After treatment as described above the cells are washed quickly with Krebs-Ringer phosphate at $37^{\circ}$C and the uptake of [3H] 2-deoxyglucose (14.2 KBq;10 mM) in Krebs Ringer phosphate at $37^{\circ}$C with or without $10^{-7}$M insulin over a 10 minute period is then measured. The reaction is stopped by aspiration of the glucose solution and rapid washing with ice cold phosphate buffered saline. Cells are lysed by the addition of 0.2M sodium hydroxide and the solution is neutralised by the addition of 0.2M hydrocholoric acid prior to scintillation counting in Optiphase scintillant using a Wallac 1410 liquid scintillation counter.

EXAMPLE 7

In another example of the invention Domains E and T are produced from the same serotype of botulinum neurotoxin and are produced already coupled together. The neurotoxin, serotype A, of *C. botulinum* is subjected to limited proteolysis using tosylphenylalaninechloromethane-treated trypsin. The E-T complex is then purified by chromatography on Sephadex G-200 (Shone, CC; Hambleton, P; & Melling, J 1985, Eur. J. Biochem. 151, 75-82).

It should be noted that this fragment is equivalent to that referred to as the $H_2L$ fragment. Any remaining free sulphydryls are derivatized by the addition of 150mM iodoacetamide as described in example 6. The binding Domain (B) is insulin-like growth factor II prepared as described in Example 6, and coupled to the E-T complex using SPDP as described. The activity of the agent on the expression of insulin-dependent glucose transport in adipocytes is tested as described in Example 6.

EXAMPLE 8

In another example of the invention, an agent for the regulation of the cell surface expression of the CR1 receptor for complement fragment C3b in neutrophils (CD 35) is synthesized in the following manner. The B Domain is prepared from the SHCL3 monoclonal antibody to the leukocyte adhesion molecule P150,95. The E and T Domains are prepared from botulinum neurotoxin, serotype A, ready coupled, as described in Example 7, and are coupled to Domain B, as described in that example.

The preferred method for testing the activity of the agent on neutrophil cell surface expression of CR1 (CD35) is using the whole blood lysing technique. EDTA anticoagulated whole blood from normal donors is treated with the agent for 4 hours and then activated for 30 minutes at $37°C$ using $10^{-6}M$ fMet-Leu-Phe diluted in PBS from a stock of $10^{-2}M$ made up in DMSO. Control cells are incubated with PBS. The blood is then incubated for 30 minutes at room temperature with 10ml of Phycoerythrin conjugated monoclonal antibody antiCD35 (Serotec:MCA 554PE), red blood cells are lysed using Becton Dickinson lysing fluid, leukocytes washed with PBS and resuspended in 2% formaldehyde in PBS. Suface bound PE is analysed by flow cytometry using a FACScan (Becton Dickinson) equipped with Lysys II software.

EXAMPLE 9

In another example of the invention, an agent for the regulation of the cell surface expression of the leukocyte adhesion molecule Mac-1 (CD IIb) is synthesized in the following manner. The B Domain is prepared from the SHCL3 monoclonal antibody to the leukocyte adhesion molecule P150,95 by standard methodologies using pepsin, and is purified by gel filtration (Martin, FJ; Hubbell, WL; and Papahadjopoulos, D, 1981, Biochemistry 20, 4229-4238). The E and T Domains are prepared from botulinum neurotoxin, serotype A, ready coupled, as described in Example 7, and are coupled to Domain B as described in that example.

The preferred method for testing the activity of the agent on neutrophil cell surface expression of Mac-1 (CD11b) is using the whole blood lysing technique. EDTA anticoagulated whole blood from normal donors is treated with the agent for 4 hours at $37°C$ and then activated for 30 minutes at $37°C$ using $10^{-6}M$ fMet-Leu-Phe diluted in PBS from a stock of $10^{-2}M$ made up in DMSO. Control cells are incubated with PBS. The blood is then incubated for 30 minutes at room temperature with 10ml of fluoroscein isothiocyanate conjugated monoclonal antibody antiCD11b (Serotec:MCA 551F). The red blood cells are lysed using Becton Dickinson lysing fluid, the leukocytes washed with PBS and resuspended in 2% formaldehyde in PBS. Surface bound FITC is analysed by flow cytometry using a FACScan (Becton Dickinson) equipped with Lysys II software.

EXAMPLE 10

In another example of the invention, an agent for the regulation of the cell surface content of $Na^+$ channels in the apical membrane of bladder epithelium is synthesized in the following manner. The B Domain is prepared from a high affinity monoclonal antibody to a cell surface marker of bladder epithelial cells by standard methodology using pepsin, and is purified by gel filtration (Martin, FJ; Hubbell, WL; and Papahadjopoulos, D, 1981, Biochemistry 20, 4229-4238). The E and T Domains are prepared from botulinum neurotoxin, serotype A, ready coupled, as described in Example 7, and are coupled to Domain B as described in that example.

The effect of the agent on aldosterone-stimulated increases in amiloride-sensitive $Na^+$- channels is tested using urinary epithelial cells. Bladder epithelial cells, prepared as primary cultures from rat bladder (Johnson, MD; Bryan, GT; Reznikoff, CA;1985, J Urol <u>133</u>, 1076-1081), are incubated with or without the agent for 90 mins at 37°C. Aldosterone-treated cells are then exposed for 1h to aldosterone. After treatment as described, the cells are rapidly washed and the amiloride-sensitive uptake of $^{22}Na^+$ over a 5 min incubation at 37°C is measured.

EXAMPLE 11

In another example of the invention, an agent for regulating antigen-presentation by B-cells is synthesized in the following manner. The B Domain is prepared from the pan B cell monoclonal antibody LL2 using standard methodology using pepsin, and is purified by gel filtration (Martin, FJ; Hubbell, WL & Papahadjopoulos, D, 1981, Biochemistry, <u>20</u>, 4229-4238). The E and T Domains are prepared from botulinum neurotoxin, serotype A, ready coupled, as described in Example7, and are coupled to Domain B also as described in that example.

The effect of the agent on antigen-presentation is tested using the murine B lymphoma cell-line TA3. These cells are first incubated with the agent for 90 mins at 37°C, and then hen egg lysozyme (HEL) is added and the incubation continued for 2h at 37°C. The TA3 cells are then fixed and washed before culture with the I-A$^K$- restricted HEL46-61 specific T-cell hybridoma 3A9 (Lorenz, RG & Alien, PM, 1989, Nature <u>337</u>, 560). The supernatant from the 3A9 cells is tested for its ability to support growth of the IL-2-dependent cell line, CTLL. Proliferative responses are measured by the incorporation of $^3$H-thymidine over a 3h period following 2 days of culture with the supernatant.

The examples described above are purely illustrative of the invention. It should be clear to those skilled in the art that any combination of the three domains are within the scope of this invention. In synthesising the agent the coupling of the T-E component of the invention to the targeting component is achieved via chemical coupling using reagents and techniques known to those skilled in the art. Thus, although the examples given use exclusively the SPDP coupling reagent any other coupling reagent capable of covalently attaching the targeting component of the reagent and known to those skilled in the art is covered in the scope of this application. Similarly it is evident to those skilled in the art that either the DNA coding for either the entire agent or fragments of the agent could be readily constructed and, when expressed in an appropriate organism, could be used to produce the agent or fragments of the agent. Such genetic constructs of the agent of the invention obtained by techniques known to those skilled in the art are also covered in the scope of this invention.

EXPLOITATION IN INDUSTRY

The agent described in this invention can be used *in vivo* either directly or as a pharmaceutically acceptable salt or ester in a method of treatment for a variety of pathophysiological states.

For example, one form of the agent can be used in a method of treatment for glucose metabolism disorders by limiting the uptake of glucose by certain cells. A specific example of this would be the use of a form of the agent in a method of treatment for clinical obesity by limiting the uptake of glucose by adipose cells and hence reducing accumulation of lipid in these cells.

In another example a form of the agent can be used for the treatment of hypertension by regulating the ion uptake by kidney cells and hence controlling the output of fluid from these organs.

In yet another example a form of the agent can be used for the treatment of inflammation by controlling the response of target cells to external signals which trigger the inflammatory response.

In yet another example a form of the agent can be used for the treatment of immune disorders by controlling the presentation of peptide sequences by antigen presenting cells to the effector cells of the immune system.

TABLE 1

| IGF-1 Up-Regulation Of $^{125}$I-Transferrin Binding In 3T3-LI Fibroblasts | | |
|---|---|---|
| Treatment | IGF-1 | % basal binding ± SD* |
| Control | - | 100 ± 28 (n=3) |
|  | + | 258 ± 46 (n=3) |
| BoNT-B | - | 100 ± 8 (n=3) |
|  | + | 149 ± 27 (n=3) |

* Specific binding of $^{125}$I-transferrin to 3T3-LI fibroblasts expressed as a percentage of the specific binding in the absence of IGF-1.

TABLE 2

| IGF-1 Up-regulation of $^{125}$I-transferrin binding in 3T3-LI fibroblasts | | |
|---|---|---|
| Treatment | IGF-1 | % basal binding ± SD* |
| Control | - | 100 ± 28 (n=3) |
| | + | 258 ± 46 (n=3) |
| BoNT-A | - | 100 ± 44 (n=3) |
| | + | 149 ± 10 (n=3) |

* Specific binding of $^{125}$I-transferrin to 3T3-LI fibroblasts expressed as a percentage of the specific binding in the absence of IGF-1.

TABLE 3

| IGF-1 Up regulation of $^{125}$I-transferrin binding in 3T3-LI fibroblasts | | |
|---|---|---|
| Treatment | IGF-1 | % basal binding ± SD* |
| Control | - | 100 ± 28 (n=3) |
| | + | 258 ± 46 (n=3) |
| $H_2$L-A | - | 100 ± 15 (n=3) |
| | + | 134 ± 60 (n=3) |

* Specific binding of $^{125}$I-transferrin to 3T3-LI fibroblasts expressed as a percentage of the specific binding in the absence of IGF-1.

TABLE 4

| Uptake of [$^3$H] -2-deoxyglucose by 3T3-LI adipocytes | | |
|---|---|---|
| | Basal | Insulin-stimulated |
| Control | 1655 ± 67 (n=3) | 14 328 ± 264 (n=3) |
| BoNT-A treated | 2306 ± 49 (n=3) | 5587 ± 322 (n=3) |

The results are the means ± SEM of triplicate determinations and are given as the total dpm taken up by the cell monolayer during a 10 min incubation.

## Claims

1. An agent for controlling the interaction of a cell with its external environment by controlling the transport of Integral Membrane Proteins (IMPs) to the membrane of the cell in Recyclable Membrane Vesicles (RMVs) which comprises three Domains B, T and E,
Where

   Domain B is a binding Domain which binds the agent to a Binding Site on the cell which undergoes endocytosis to be incorporated into an endosome, where Domain B is not the native binding Domain of a Clostridial neutotoxin

   Domain T is a Translocation Domain that translocates Domain E (with or without other domains of the agent and/or the Binding Site) from within the endosome across the endosomal membrane into the cytosol of the cell

   Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity; with the proviso that the agent is not the disulphide-linked conjugate $I_{bc}$-ricin B chain or the disulphide-linked conjugate $I_{bc}$-wheat germ agglutinin, wherein $I_{bc}$ is obtained by enzymic treatment of tetanus toxin with papain.

2. An agent according to Claim 1 in which Domain T is a domain or domain fragment of Clostridial neurotoxin Heavy Chain.

3. An agent according to Claim 1 or 2 in which Domain E is obtained from botulinum neurotoxin.

4. An agent according to any preceding claim in which Domain E is obtained from botulinum neurotoxin type A.

5. An agent according to claim 1 to 3 in which Domain E is obtained from botulinum neurotoxin type B.

6. An agent according to any preceding claim in which Domain T is the amino terminal portion of the heavy chain of a Clostridial neurotoxin.

7. An agent according to any preceding claim in which Domain T is the amino terminal portion of the heavy chain of a Clostridial neurotoxin obtained by proteolytic cleavage with trypsin.

8. An agent according to any preceding Claim in which Domain T is obtained from botulinum neurotoxin.

9. An agent according to any preceding claim in which Domain T is obtained from botulinum neurotoxin type A.

10. An agent according to any of claims 1-8 in which Domain T is obtained from botulinum neurotoxin type B.

11. An agent according to any preceding Claim which comprises the three Domains B, T and E, and each Domain is linked to another of said Domains by X,
where X is either a covalently linked spacer molecule or a direct covalent linkage and at least one X is a spacer molecule.

12. An agent according to any preceding Claim in which Domain B comprises a ligand to a cell surface marker on the target cell capable of undergoing endocytosis to be incorporated into an endosome.

13. An agent according to any preceding Claim in which Domain B comprises a ligand to a cell surface receptor on the target cell capable of undergoing endocytosis to be incorporated into an endosome.

14. An agent according to any preceding claim in which Domain B comprises a monoclonal antibody or is derived from a monoclonal antibody to a surface antigen on the target cell capable of undergoing endocytosis to be incorporated into an endosome.

15. An agent according to any preceding Claim in which Domain B, the Binding Domain, binds to a Binding Site which is characteristic of a particular cell type.

16. An agent according to any preceding claim in which domain B is a ligand to the insulin like growth factor II receptor.

17. An agent according to claim 16 in which Domain B is insulin like growth factor II.

18. An agent according to any one of claims 1 to 15 in which Domain B is, or is derived from, the monoclonal antibody SHCL3.

19. An agent according to any one of claims 1 to 15 in which Domain B is, or is derived from, monoclonal antibody LL2.

20. An agent according to claim 17 in which Domain B is insulin like growth factor II, Domain T is the amino terminal portion of the heavy chain of botulinum neurotoxin type A obtained by proteolytic cleavage using trypsin, and Domain E is the light chain of botulinum neurotoxin type A.

21. An agent according to claim 18 in which Domain B is ' the monoclonal antibody SHCL3, Domain T is the amino terminal portion of the heavy chain of botulinum neurotoxin type A obtained by proteolytic cleavage using trypsin, and Domain E is the light chain of botulinum neurotoxin type A.

22. An agent according to claim 19 in which Domain B is the monoclonal antibody LL2, Domain T is the amino-terminal portion of the heavy chain of botulinum neurotoxin type A obtained by proteolytic cleavage using trypsin, and Domain E is the light chain of botulinum neurotoxin type A.

23. A method for obtaining an agent according to any preceding claim which comprises the covalent attachment of the three Domains B, T and E.

24. A method for obtaining an agent according to any one of claims 1 to 22 which comprises the covalent attachment of the three Domains B, T and E wherein each Domain is linked to another of said Domain by X,
where X is either a covalently linked spacer molecule or a direct covalent linkage and at least one X is a spacer molecule.

25. A method of obtaining the agent according to any one of Claims 1 to 22 which comprises constructing a genetic construct which codes for the agent, incorporating said construct into a host organism and expressing the construct to produce the agent.

26. Use of an agent which comprises three Domains B, T and E,
Where

    Domain B is a binding Domain which binds the agent to a Binding Site on the cell which undergoes endocytosis to be incorporated into an endosome, where Domain B is not the native binding Domain of a Clostridial neutotoxin

    Domain T is a Translocation Domain that translocates Domain E (with or without other domains of the agent and/or the Binding Site) from within the endosome across the endosomal membrane into the cytosol of the cell

    Domain E is the Effector Domain which inhibits the ability of the RMVs to transport the IMPs to the surface of the cell and is a domain or domain fragment of the Light chain of Clostridial neurotoxin having $Zn^{2+}$ dependent

metalloprotease activity in the manufacture of a medicament for controlling the interaction of a cell with its external environment by controlling the transport of IMPs by RMVs to a cell membrane.

27. Use of an agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity in the manufacture of a medicament for controlling the level of an IMP responsible for transport of a metabolite across a cell membrane.

28. Use of an agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity in the manufacture of a medicament for controlling the level of an IMP responsible for selective permeability of a plasma membrane to an ion.

29. Use of an agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity

in the manufacture of a medicament for controlling the level of an IMP responsible for recognition of a signalling molecule.

30. Use of an agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity in the manufacture of a medicament for controlling the level of an IMP responsible for the transduction of a signal across a cell membrane following binding to the membrane of a signalling molecule.

31. Use of an agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity in the manufacture of a medicament for controlling the level of an IMP responsible for the display on a cell surface of a peptide fragment derived from an ingested antigen.

32. The agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity or a pharmaceutically acceptable salt thereof for use as a medicament for the treatment of glucose metabolism disorders.

33. The agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity or a pharmaceutically acceptable salt thereof for use as a medicament for the treatment of clinical obesity.

34. The agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity or a pharmaceutically acceptable salt thereof for use as a medicament for the treatment of hypertension.

35. The agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity or a pharmaceutically acceptable salt thereof for use as a medicament for the treatment of inflammatory disorders.

36. The agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity or a pharmaceutically acceptable salt thereof for use as a medicament for the treatment of immune system disorders.

37. Use of the agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity in the manufacture of a medicament for the treatment of glucose metabolism disorders.

38. Use of the agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity in the manufacture of a medicament for the treatment of clinical obesity.

39. Use of the agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity in the manufacture of a medicament for the treatment of hypertension.

40. Use of the agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity in the manufacture of a medicament for the treatment of inflammatory disorders.

41. Use of the agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity in the manufacture of a medicament for the treatment of immune system disorders.

42. A pharmaceutical composition which comprises an effective amount of the agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity for treatment of glucose metabolism disorders.

43. A pharmaceutical composition which comprises an effective amount of the agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity for treatment of clinical obesity.

44. A pharmaceutical composition which comprises an effective amount of the agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity for treatment of hypertension.

45. A pharmaceutical composition which comprises an effective amount of the agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity for treatment of inflammatory disorders.

46. A pharmaceutical composition which comprises an effective amount of the agent which comprises three Domains B, T and E,
Where

Domain B is a binding Domain which binds the agent to a Binding Site on the cell which undergoes endocytosis to be incorporated into an endosome, where Domain B is not the native binding Domain of a Clostridial neutotoxin

Domain T is a Translocation Domain that translocates Domain E (with or without other domains of the agent and/or the Binding Site) from within the endosome across the endosomal membrane into the cytosol of the cell

Domain E is the Effector Domain which inhibits the ability of the RMVs to transport the IMPs to the surface of the cell and is a domain or domain fragment of the Light chain of Clostridial neurotoxin having $Zn^{2+}$ dependent metalloprotease activity for treatment of immune system disorders.

**Patentansprüche**

1. Mittel für die Steuerung der Interaktion einer Zelle mit ihrer äußeren Umgebung durch Steuern des Transports von integralen Membranproteinen (IMP) zur Membran der Zelle in recycelbaren Membranvesikeln (RMV), das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,
die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,
die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt, mit der Maßgabe, dass das Mittel nicht das disulfid-verbrückte Konjugat von $I_{bc}$-Ricin B-Kette oder das disulfid-verbrückte Konjugat von $I_{bc}$-Weizenkeimagglutinin ist, worin das $I_{bc}$ durch Enzymbehandlung des Tetanus-Toxins mit Papain erhalten wird.

2. Mittel gemäß Anspruch 1, worin Domäne T eine Domäne oder Domänenfragment der schweren Kette eines Clostridien-Neurotoxins ist.

3. Mittel gemäß Anspruch 1 oder 2, worin Domäne E aus Botulinus-Neurotoxin erhalten wird.

4. Mittel gemäß einem der vorstehenden Ansprüche, worin die Domäne E aus dem Botulinus-Neurotoxintyp A erhalten wird.

5. Mittel gemäß Anspruch 1 bis 3, worin die Domäne E aus Botulinus-Neurotoxintyp B erhalten wird.

6. Mittel gemäß einem der vorstehenden Ansprüche, worin Domäne T der amino-terminale Teil der schweren Kette eines Clostridien-Neurotoxins ist.

7. Mittel gemäß einem der vorstehenden Ansprüche, worin die Domäne T der amino-terminale Teil der schweren Kette eines Clostridien-Neurotoxins ist, erhalten durch proteolytische Spaltung mit Trypsin.

8. Mittel gemäß einem der vorstehenden Ansprüche, worin die Domäne T aus Botulinus-Neurotoxin erhalten wird.

9. Mittel gemäß einem der vorstehenden Ansprüche, worin die Domäne T aus Botulinus-Neurotoxintyp A erhalten wird.

10. Mittel gemäß einem der vorstehenden Ansprüche 1 bis 8, worin die Domäne T aus Botulinus-Neurotoxintyp B erhalten wird.

11. Mittel gemäß einem der vorstehenden Ansprüche, das die drei Domänen B, T und E enthält und worin jede Domäne an eine andere der Domänen mit Hilfe von X gebunden ist, worin X entweder ein kovalent gebundenes Spacermolekül oder eine direkte kovalente Bindung darstellt und worin mindestens ein X ein Spacermolekül ist.

12. Mittel gemäß einem der vorstehenden Ansprüche, worin die Domäne B einen Liganden zu einem Zelloberflächenmarker auf der Zielzelle umfasst, befähigt zur Endocytose zur Inkorporation in ein Endosom.

**13.** Mittel gemäß einem der vorstehenden Ansprüche, worin die Domäne B einen Liganden an einen Zelloberflächenrezeptor auf der Zielzelle umfasst, befähigt zur Endocytose zur Inkorporation in ein Endosom.

**14.** Mittel gemäß einem der vorstehenden Ansprüche, worin die Domäne B einen monoklonalen Antikörper umfasst oder von einem monoklonalen Antikörper gegen ein Oberflächenantigen auf der Zielzelle abgeleitet ist, befähigt zur Endocytose zur Inkorporation in ein Endosom.

**15.** Mittel gemäß einem der vorstehenden Ansprüche, worin die Domäne B, die Bindungsdomäne, an eine Bindungsstelle bindet, die für einen speziellen Zelltyp charakteristisch ist.

**16.** Mittel gemäß einem der vorstehenden Ansprüche, worin die Domäne B ein Ligand für den Rezeptor des Insulinlike-Growthfactors II (IGF-II-Rezeptor) ist.

**17.** Mittel gemäß Anspruch 16, worin die Domäne B der Insulinlike-Growthfactor II (IGF-II) ist.

**18.** Mittel gemäß einem der Ansprüche 1 bis 15, worin die Domäne B der monoklonale Antikörper SHCL3 ist oder von diesem abgeleitet ist.

**19.** Mittel gemäß einem der Ansprüche 1 bis 15, worin die Domäne B der monoklonale Antikörper LL2 ist oder von diesem abgeleitet ist.

**20.** Mittel gemäß Anspruch 17, worin die Domäne B der Insulinlike-Growthfactor II (IGF-II) ist, die Domäne T den amino-terminalen Teil der schweren Kette des Botulinus-Neurotoxintyps A darstellt, erhalten über proteolytische Spaltung unter Verwendung von Trypsin, und worin die Domäne E die leichte Kette des Botulinus-Neurotoxintyps A darstellt.

**21.** Mittel gemäß Anspruch 18, worin die Domäne B der monoklonale Antikörper SHCL3 ist, die Domäne T den amino-terminalen Teil der schweren Kette des Botulinus-Neurotoxintyps A darstellt, erhalten durch proteolytische Spaltung unter Verwendung von Trypsin, und worin die Domäne E die leichte Kette des Botulinus-Neurotoxintyps A ist.

**22.** Mittel gemäß Anspruch 19, worin die Domäne B der monoklonale Antikörper LL2 ist, die Domäne T den amino-terminalen Teil der schweren Kette des Botulinus-Neurotoxintyps A darstellt, erhalten durch proteolytische Spaltung unter Verwendung von Trypsin, und worin die Domäne E die leichte Kette des Botulinus-Neurotoxintyps A ist.

**23.** Verfahren zum Erhalten eines Mittels gemäß einem der vorstehenden Ansprüche, das die kovalente Anbindung der drei Domänen B, T und E umfasst.

**24.** Verfahren zum Erhalten eines Mittels gemäß einem der Ansprüche 1 bis 22, das die kovalente Anheftung der drei Domänen B, T und E umfasst, worin jede Domäne an eine andere der Domänen über X gebunden ist, worin X entweder ein kovalent gebundenes Spacermolekül oder eine direkte kovalente Bindung darstellt und worin mindestens ein X ein Spacermolekül ist.

**25.** Verfahren zum Erhalten des Mittels gemäß einem der Ansprüche 1 bis 22, dass das Konstruieren eines genetischen Konstrukts, das für das Mittel kodiert, das Inkorporieren des Konstrukts in einen Wirtsorganismus und die Expression des Konstrukts zur Erzeugung des Mittels umfasst.

**26.** Verwendung eines Mittels, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,
die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,
die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt,
bei der Herstellung eines Medikaments zum Steuern der Interaktion einer Zelle mit ihrer externen Umgebung durch Steuern des Transports von IMPs durch RMVs an eine Zellmembran.

27. Verwendung eines Mittels, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,
die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,
die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt,
bei der Herstellung eines Medikaments zum Steuern des Levels an IMP, verantwortlich für den Transport eines Metaboliten über eine Zellmembran.

28. Verwendung eines Mittels, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,
die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,
die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt,
bei der Herstellung eines Medikaments zum Steuern des Levels an IMP, verantwortlich für die selektive Permeabilität einer Plasmamembran für ein Ion.

29. Verwendung eines Mittels, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,
die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,
die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt,
bei der Herstellung eines Medikaments zum Steuern des Levels eines IMP, verantwortlich für die Erkennung eines Signalmoleküls.

30. Verwendung eines Mittels, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,
die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,
die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt,
bei der Herstellung eines Medikaments zum Steuern des Levels an IMP, verantwortlich für die Übermittlung bzw. Transduction eines Signals über eine Zellmembran, folgend der Bindung eines Signalmoleküls an die Membran.

**31.** Verwendung eines Mittels, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,
die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,
die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt,
bei der Herstellung eines Medikaments zum Steuern des Levels an IMP, verantwortlich für das Präsentieren eines von einem aufgenommenen Antigen abgeleiteten Peptidfragments auf einer Zelloberfläche.

**32.** Mittel, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,
die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,
die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt,
oder pharmazeutisch annehmbares Salz desselben für die Verwendung als Medikament für die Behandlung von Störungen des Glucose-Metabolismus.

**33.** Mittel, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,
die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,
die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt,
oder pharmazeutisch annehmbares Salz desselben für die Verwendung als Medikament für die Behandlung der klinischen Fettleibigkeit.

**34.** Mittel, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,
die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,
die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt,
oder pharmazeutisch annehmbares Salz desselben für die Verwendung als Medikament für die Behandlung des Bluthochdrucks.

**35.** Mittel, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,

die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,

die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt,

oder pharmazeutisch annehmbares Salz desselben für die Verwendung als Medikament für die Behandlung von entzündlichen Erkrankungen.

**36.** Mittel, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,

die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,

die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt,

oder pharmazeutisch annehmbares Salz desselben für die Verwendung als Medikament für die Behandlung von Störungen des Immunsystems.

**37.** Verwendung des Mittels, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,

die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,

die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt,

bei der Herstellung eines Medikaments für die Behandlung von Störungen des Glucose-Metabolismus.

**38.** Verwendung des Mittels, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,

die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,

die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt,

bei der Herstellung eines Medikaments für die Behandlung der klinischen Fettleibigkeit.

**39.** Verwendung des Mittels, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,

die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,

die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt,

bei der Herstellung eines Medikaments für die Behandlung des Bluthochdrucks.

40. Verwendung des Mittels, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,

die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,

die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt,

bei der Herstellung eines Medikaments für die Behandlung von entzündlichen Erkrankungen.

41. Verwendung des Mittels, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,

die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,

die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt,

bei der Herstellung eines Medikaments für die Behandlung von Störungen des Immunsystems.

42. Pharmazeutische Zubereitung, die eine wirksame Menge eines Mittels enthält, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,

die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,

die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit $Zn^{2+}$-abhängiger Metalloprotease-Aktivität handelt,

für die Behandlung von Störungen des Glucose-Metabolismus.

43. Pharmazeutische Zubereitung, die eine wirksame Menge eines Mittels enthält, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt,

die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert,

die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit Zn$^{2+}$-abhängiger Metalloprotease-Aktivität handelt, für die Behandlung der klinischen Fettleibigkeit.

44. Pharmazeutische Zubereitung, die eine wirksame Menge eines Mittels enthält, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt, die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert, die Domäne E die Effektordomäne darstellt, die die Fähigkeit der RMVs inhibiert; die IMPs zur Oberfläche der Zelle zu transportieren, und bei der es sich um eine Domäne oder ein Domänenfragment der leichten Kette des Clostridien-Neurotoxins mit Zn$^{2+}$-abhängiger Metalloprotease-Aktivität handelt, für die Behandlung des Bluthochdrucks.

45. Pharmazeutische Zubereitung, die eine wirksame Menge eines Mittels enthält, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt, die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert, die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit Zn$^{2+}$-abhängiger Metalloprotease-Aktivität handelt, für die Behandlung von entzündlichen Erkrankungen.

46. Pharmazeutische Zubereitung, die eine wirksame Menge eines Mittels enthält, das drei Domänen B, T und E umfasst, worin

die Domäne B eine Bindungsdomäne ist, die das Mittel an eine Bindungsstelle auf der Zelle bindet, welche eine Endocytose zum Einbau in ein Endosom durchläuft, worin die Domäne B nicht die native Bindungsdomäne eines Clostridien-Neurotoxins darstellt, die Domäne T eine Translokationsdomäne darstellt, die die Domäne E (mit oder ohne andere Domänen des Mittels und/oder der Bindungsstelle) von innerhalb des Endosoms über die Endosomenmembran in das Cytosol der Zelle transloziert, die Domäne E die Effektordomäne darstellt, die die Fähigkeit der RMVs inhibiert, die IMPs zur Oberfläche der Zelle zu transportieren, und bei der es sich um eine Domäne oder ein Domänenfragment der leichten Kette des Clostridien-Neurotoxins mit Zn$^{2+}$-abhängiger Metalloprotease-Aktivität handelt, für die Behandlung von Störungen des Immunsystems.

**Revendications**

1. Agent de régulation de l'interaction d'une cellule avec son milieu externe agissant en régulant le transport de protéines membranaires intégrées (IMP) vers la membrane cellulaire à l'intérieur de Vésicules Membranaires Recyclables (RMV) qui comprend trois domaines B, T et E,

où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante ; à condition que l'agent ne soit pas le conjugué Ibc lié au disulfure - chaîne B ricine ou le conjugué Ibc lié au disulfure - agglutinine germe de froment, dans lequel Ibc est obtenu par traitement enzymatique de la toxine du tétanos avec la papaïne.

2. Agent selon la Revendication 1, dans lequel le Domaine T est un domaine ou fragment de domaine de la Chaîne Lourde de la neurotoxine de Clostridium.

3. Agent selon la revendication 1 ou 2, dans lequel le Domaine E est obtenu à partir de la neurotoxine botulique.

4. Agent selon l'une quelconque des revendications précédentes, dans lequel le Domaine E est obtenu à partir de la neurotoxine botulique de type A.

5. Agent selon l'une quelconque des revendications 1 à 3, dans lequel le Domaine E est obtenu à partir de la neurotoxine botulique de type B.

6. Agent selon l'une quelconque des revendications précédentes, dans lequel le Domaine T est la partie amino-terminale de la chaîne lourde d'une neurotoxine de Clostridium.

7. Agent selon l'une quelconque des revendications précédentes, dans lequel le Domaine T est la partie amino-terminale de la chaîne lourde d'une neurotoxine de Clostridium obtenue par clivage protéolytique avec la trypsine.

8. Agent selon l'une quelconque des revendications précédentes, dans lequel le Domaine T est obtenu à partir de la neurotoxine botulique.

9. Agent selon l'une quelconque des revendications précédentes, dans lequel le Domaine T est obtenu à partir de la neurotoxine botulique de type A.

10. Agent selon l'une quelconque des revendications précédentes, dans lequel le Domaine T est obtenu à partir de la neurotoxine botulique de type B.

11. Agent selon l'une quelconque des revendications précédentes qui comprend les trois domaines B, T et E, et chaque Domaine est lié à un autre desdits Domaines par X,

où X est soit une molécule intercalaire à liaison covalente soit une liaison covalente directe et où un X au moins est une molécule intercalaire.

12. Agent selon l'une quelconque des revendications précédentes, dans lequel le Domaine B comprend un ligand à un marqueur de surface cellulaire au niveau de la cellule cible pouvant subir une endocytose et être donc incorporé dans un endosome.

13. Agent selon l'une quelconque des revendications précédentes, dans lequel le Domaine B comprend un ligand à un récepteur de surface cellulaire présent sur la cellule cible pouvant subir une endocytose et être donc incorporé dans un endosome.

14. Agent selon l'une quelconque des revendications précédentes, dans lequel le Domaine B comprend un anticorps monoclonal ou dérive d'un anticorps monoclonal dirigé contre un antigène de surface sur la cellule cible, pouvant subir une endocytose et être donc incorporé dans un endosome.

15. Agent selon l'une quelconque des revendications précédentes, dans lequel le Domaine B, le Domaine de Liaison, se fixe à un Site de Liaison qui est caractéristique d'un type cellulaire donné.

16. Agent selon l'une quelconque des revendications précédentes, dans lequel le domaine B est un ligand du récepteur du facteur de croissance analogue à l'insuline II.

17. Agent selon la revendication 16, dans lequel le Domaine B est le facteur de croissance analogue à l'insuline II.

18. Agent selon l'une quelconque des revendications 1 à 15, dans lequel le Domaine B est l'anticorps monoclonal SHCL3 ou en dérive.

19. Agent selon l'une quelconque des revendications 1 à 15, dans lequel le Domaine B est de l'anticorps monoclonal LL2 ou en dérive.

20. Agent selon la revendication 17, dans lequel le domaine B est le facteur de croissance analogue à l'insuline II, le Domaine T est la partie amino-terminale de la chaîne lourde de la neurotoxine botulique de type A obtenue par clivage protéolytique avec la trypsine, et le Domaine E est la chaîne légère de la neurotoxine botulique de type A.

21. Agent selon la revendication 18, dans lequel le Domaine B est l'anticorps monoclonal SHCL3, le Domaine T est la partie amino-terminale de la chaîne lourde de la neurotoxine botulique de type A obtenue par clivage protéolytique avec la trypsine, et le Domaine E est la chaîne légère de la neurotoxine botulique de type A.

22. Agent selon la revendication 19, dans lequel le Domaine B est l'anticorps monoclonal LL2, le Domaine T est la partie amino-terminale de la chaîne lourde de la neurotoxine botulique de type A obtenue par clivage protéolytique avec la trypsine, et le Domaine E est la chaîne légère de la neurotoxine botulique de type A.

23. Procédé d'obtention d'un agent selon l'une quelconque des revendications précédentes qui comprend la jonction covalente des trois Domaines B, T et E.

24. Procédé d'obtention d'un agent selon l'une quelconque des revendications 1 à 22 qui comprend la jonction covalente des trois Domaines B, T et E, dans lequel chaque domaine est lié à un autre desdits Domaines par X,
où X est soit une molécule intercalaire à liaison covalente soit une liaison covalente directe et où un X au moins est une molécule intercalaire.

25. Procédé d'obtention de l'agent selon l'une quelconque des revendications 1 à 22 qui comprend la réalisation d'une construction génétique qui code pour ledit agent, l'incorporation de ladite construction dans un organisme hôte et l'expression de la construction pour produire l'agent.

26. Utilisation d'un agent qui comprend trois domaines B, T et E,
où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium
le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique
le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $2n^{2+}$-dépendante dans l'obtention d'un médicament pour réguler l'interaction d'une cellule avec son milieu externe qui agit en régulant le transport des IMP par RMV vers la membrane cellulaire.

27. Utilisation d'un agent qui comprend trois domaines B, T et E,
où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium
le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique
le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante dans l'obtention d'un médicament pour réguler le taux d'IMP responsable du transport d'un métabolite à travers la membrane cellulaire.

**28.** Utilisation d'un agent qui comprend trois domaines B, T et E,

où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante dans l'obtention d'un médicament pour réguler le taux d'IMP responsable de la perméabilité sélective d'une membrane plasmique à un ion.

**29.** Utilisation d'un agent qui comprend trois domaines B, T et E,

où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante dans l'obtention d'un médicament pour réguler le taux d'IMP responsable de la reconnaissance d'une molécule de signalisation.

**30.** Utilisation d'un agent qui comprend trois domaines B, T et E,

où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante dans l'obtention d'un médicament pour réguler le taux d'IMP responsable de la transduction d'un signal à travers la membrane cellulaire suite à la liaison d'une molécule de signalisation à cette membrane.

**31.** Utilisation d'un agent qui comprend trois domaines B, T et E,

où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante dans l'obtention d'un médicament pour réguler le taux d'IMP responsable de la présentation d'un fragment peptidique dérivé d'un antigène ingéré à la surface cellulaire.

**32.** Agent qui comprend trois domaines B, T et E,

où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante ou un sel pharmaceutiquement acceptable correspondant pour utilisation comme médicament dans le traitement des troubles de métabolisme du glucose.

**33.** Agent qui comprend trois domaines B, T et E,

où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante ou un sel pharmaceutiquement acceptable correspondant pour utilisation comme médicament dans le traitement de l'obésité clinique.

**34.** Agent qui comprend trois domaines B, T et E,

où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante ou un sel pharmaceutiquement acceptable correspondant pour utilisation comme médicament dans le traitement de l'hypertension.

**35.** Agent qui comprend trois domaines B, T et E,

où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante ou un sel pharmaceutiquement acceptable correspondant pour utilisation comme médicament dans le traitement des troubles inflammatoires.

**36.** Agent qui comprend trois domaines B, T et E,

où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante ou un sel pharmaceutiquement acceptable correspondant pour utilisation comme médicament dans le traitement des troubles du système immunitaire.

**37.** Utilisation de l'agent qui comprend trois domaines B, T et E,
   où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante dans l'obtention d'un médicament pour le traitement des troubles de métabolisme du glucose.

**38.** Utilisation de l'agent qui comprend trois domaines B, T et E,
   où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante dans l'obtention d'un médicament pour le traitement de l'obésité clinique.

**39.** Utilisation de l'agent qui comprend trois domaines B, T et E,
   où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante dans l'obtention d'un médicament pour le traitement de l'hypertension.

**40.** Utilisation de l'agent qui comprend trois domaines B, T et E,
   où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante dans l'obtention d'un médicament pour le traitement des troubles inflammatoires.

41. Utilisation de l'agent qui comprend trois domaines B, T et E,
où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante dans l'obtention d'un médicament pour le traitement des troubles du système immunitaire.

42. Composition pharmaceutique qui comprend une quantité efficace de l'agent qui comprend trois domaines B, T et E,
où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante pour le traitement des troubles de métabolisme du glucose.

43. Composition pharmaceutique qui comprend une quantité efficace de l'agent qui comprend trois domaines B, T et E,
où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante pour le traitement de l'obésité clinique.

44. Composition pharmaceutique qui comprend une quantité efficace de l'agent qui comprend trois domaines B, T et E,
où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante pour le traitement de l'hypertension.

**45.** Composition pharmaceutique qui comprend une quantité efficace de l'agent qui comprend trois domaines B, T et E, où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domaines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante pour le traitement des troubles inflammatoires.

**46.** Composition pharmaceutique qui comprend une quantité efficace de l'agent qui comprend trois domaines B, T et E, où

le Domaine B est un Domaine de liaison fixant l'agent à un Site de Liaison au niveau de la cellule pouvant subir une endocytose et être donc incorporé dans un endosome, où le Domaine B n'est pas le Domaine de Liaison natif d'une neurotoxine de Clostridium

le Domaine T est un Domaine de Translocation qui opère la translocation du Domaine E (avec ou sans les autres domcines de l'agent et/ou du Site de Liaison) à partir de l'endosome vers le cytosol de la cellule à travers la membrane endosomique

le Domaine E est le Domaine Effecteur qui inhibe la capacité des RMV à transporter les IMP jusqu'à la surface cellulaire et consiste en un domaine ou fragment de domaine de la chaîne légère de la neurotoxine de Clostridium à activité métalloprotéase $Zn^{2+}$-dépendante pour le traitement des troubles du système immunitaire.

SEQUENCE LISTING

(1) GENERAL INFORMATION:

    (i) APPLICANT:

        (A) NAME: THE SPEYWOOD LABORATORY LIMITED
        (B) STREET: ST GEORGE'S HOSPITAL MEDICAL SCHOOL, CRAMMER TERRACE
        (C) CITY: LONDON
        (E) COUNTRY: UNITED KINGDOM
        (F) POSTAL CODE (ZIP): SM17 0QS

        (A) NAME: PUBLIC HEALTH LABORATORY SERVICE BOARD
        (B) STREET: 61 COLINDALE AVENUE
        (C) CITY: LONDON
        (E) COUNTRY: UNITED KINGDOM
        (F) POSTAL CODE (ZIP): NW9 5DF

        (A) NAME: DR. JOHN NORTH
        (B) STREET: THE OLD VICARAGE, CHURCH STREET
        (C) CITY: AMESBURY

(D) STATE: WILTSHIRE
(E) COUNTRY: UNITED KINGDOM
(F) POSTAL CODE (ZIP): SP4 7EU

(A) NAME: DR. KEITH ALAN FOSTER
(B) STREET: 137 OAKS AVENUE
(C) CITY: WORCESTER PARK
(D) STATE: SURREY
(E) COUNTRY: UNITED KINGDOM
(F) POSTAL CODE (ZIP): KT4 SXG

(A) NAME: CONRAD PADRAIG QUINN
(B) STREET: 36 ST FRANCIS ROAD
(C) CITY: SALISBURY
(D) STATE: WILTSHIRE
(E) COUNTRY: UNITED KINGDOM
(F) POSTAL CODE (ZIP): SP1 3QS

(A) NAME: CLIFFORD CHARLES SHONE
(B) STREET: 44 OAKWOOD GROVE
(C) CITY: ALDERBURY
(D) STATE: WILTSHIRE
(E) COUNTRY: UNITED KINGDOM
(F) POSTAL CODE (ZIP): SP5 3BN

(ii) TITLE OF INVENTION: NOVEL AGENT FOR CONTROLLING CELL ACTIVITY

(iii) NUMBER OF SEQUENCES: 1

(iv) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0. Version #1.25 (EPO)

(vi) PRIOR APPLICATION DATA:

(A) APPLICATION NUMBER: GB 9305735.4
(B) FILING DATE: 19-MAR-1993

(2) INFORMATION FOR SEQ ID NO: 1:

(i) SEQUENCE CHARACTERISTICS:

(A) LENGTH: 62 amino acids
(B) TYPE: amino acid
(D) TOPOLOGY: linear

(ii) MOLECULE TYPE: peptide
(xi) SEQUENCE DESCRIPTION: SEQ ID NO: 1:

```
Gln Gln Thr Gln Ala Gln Val Asp Glu Val Val Asp Ile Met Arg Val
1               5                   10          15

Asn Val Asp Lys Val Leu Glu Arg Asp Gln Lys Leu Ser Glu Leu Asp
            20              25              30

Asp Arg Ala Asp Ala Leu Gln Ala Gly Ala Ser Gln Phe Glu Thr Ser
        35              40              45

Ala Ala Lys Leu Lys Arg Lys Tyr Trp Trp Lys Asn Leu Lys
        50              55              60
```